# FASCICULE DE BREVET EUROPEEN

(11) **EP 1 191 912 B1**
(45) Date de publication et mention de la délivrance du brevet: **23.04.2003**
(21) Numéro de dépôt: 00931314.9
(22) Date de dépôt: 19.05.2000
(51) Int. Cl.: A61F 13/06, A61F 13/10

(54) **MANCHON ELASTIQUE A GARNITURE VISCOELASTIQUE POUR LA PROTECTION OU LE SOIN DE DOIGTS OU D'ORTEILS**
ELASTISCHE HÜLSE MIT EINEN VISKOELASTISCHEN POLSTER ZUM SCHUTZ ODER PFLEGE DER FINGER ODER ZEHEN
ELASTIC SLEEVE WITH A VISCOELASTIC LINING FOR THE PROTECTION OR CARE OF FINGERS OR TOES

(30) Priorité: 21.05.1999 FR 9906828
(43) Date de publication de la demande: 03.04.2002
(73) Titulaire: Epitact MD, 26270 Loriol sur Drome (FR)
(72) Inventeur: MARTIN, Jean-Luc, F-26270 Loriol (FR)
(74) Mandataire: Marchand, André
(86) Numéro de dépôt international: FR0001359
(87) Numéro de publication internationale: WO00071066

(56) Documents cités:
- CH-A- 174 207
- FR-A- 723 788
- FR-A- 2 681 780
- GB-A- 476 595
- US-A- 2 461 872
- US-A- 2 646 795
- US-A- 2 932 295
- US-A- 4 150 442
- US-A- 4 315 504
- US-A- 5 497 789

## Description

La présente invention concerne le domaine des pansements en forme de manchon pour la protection et le soin des doigts, des orteils et, plus généralement, de membres du corps humain.

Les extrémités des membres du corps humain sont exposées à de nombreuses sollicitations donnant lieu à des blessures ou à des affections qui nécessitent des moyens de protection ou de soin.

Il existe à cet effet diverses structures et formes variées de manchons comportant des matériaux viscoélastiques. On connaît notamment des manchons tubulaires constitués uniquement de polymères viscoélastiques. L'inconvénient de ces manchons est que le tube polymère doit rassembler des propriétés mécaniques d'élasticité, de résistance, de viscoélasticité et d'encombrement réduit qui sont contradictoires et ne sont pas combinés de manière satisfaisante au détriment des effets physiologiques du manchon. On connaît également des manchons tissés comprenant une garniture de matériau viscoélastique. Ces manchons ont pour inconvénient de présenter un fort encombrement, le tissu et la garniture devant avoir une épaisseur importante du fait des mauvaises propriétés mécaniques des matériaux utilisés. En outre, pour résoudre le problème de réalisation industrielle d'un manchon tubulaire en tissu, on fait appel à des tissus tricotés à mailles larges au détriment de l'épaisseur et de la tenue du manchon ainsi que du confort de l'utilisateur.

Ainsi, un objectif de la présente invention est de réaliser un manchon de protection ou de soin de doigt ou d'orteil qui présente une optimisation des paramètres de protection et de confort.

Plus particulièrement, un objectif de la présente invention est de prévoir un manchon de protection qui soit de faible épaisseur tout en offrant une résistance élevée, qui offre une grande souplesse, une bonne tenue en place sur un doigt ou un orteil, une bonne adaptabilité aux différentes tailles de doigts ou d'orteils et une excellente compatibilité avec la peau y compris en cas de lésion.

Un objectif essentiel de la présente invention est de minimiser l'encombrement et la gêne d'un manchon sans nuire à son efficacité.

Un objectif particulier de la présente invention est de prévoir un manchon offrant une protection se substituant à celle de l'épiderme, c'est-à-dire apte à absorber les frottements et à répartir les contraintes mécaniques.

Un autre objectif de la présente invention est de réaliser un manchon lavable et réutilisable sans perte de ses qualités.

Encore un autre objectif de la présente invention est de prévoir un procédé de fabrication collective de manchons à faible prix de revient.

Pour atteindre ces objectifs, une idée de la présente invention est de réaliser tout ou partie du corps d'un manchon du type précité au moyen d'un tissu comprenant des fibres thermoplastiques, offrant des caractéristiques avantageuses de soudabilité à chaud (thermosoudure), de faible épaisseur et de résistance mécanique tout en étant lavable. De préférence, un tel tissu comprend également des fibres élastiques pour l'obtention de l'élasticité nécessaire au maintien du manchon sur un doigt ou un orteil. D'autre part, une autre idée de la présente invention est de revêtir tout ou partie de la surface interne ou externe d'un tel manchon avec un gel viscoélastique, de préférence un gel silicone ayant des propriétés mécaniques similaires au capiton plantaire humain, afin de protéger idéalement contre le stress épidermique.

Plus particulièrement, la présente invention prévoit un manchon de protection ou de soin de doigts, orteils ou autres parties du corps, comprenant au moins une pièce de tissu comprenant des fibres themoplastiques, assemblée par soudage le long de ses bords pour former tout ou partie du corps du manchon, et au moins une couche de gel viscoélastique agencée sur une face de la pièce de tissu.

Selon un mode de réalisation, le manchon comprend deux pièces de tissu soudées ensemble le long de deux bords, l'une des pièces de tissu portant la couche de gel viscoélastique, les zones de soudure des deux pièces de tissu étant en retrait des limites de la couche de gel.

Selon un mode de réalisation, au moins une des deux pièces de tissu comprend des fibres élastiques.

Selon un mode de réalisation, le manchon comprend une pièce de tissu repliée comprenant des fibres élastiques et des fibres thermoplastiques, ayant deux bords opposés soudés et portant sur l'une de ses faces la couche de gel viscoélastique.

Selon un mode de réalisation, le gel viscoélastique est un gel silicone.

Selon un mode de réalisation, le gel silicone présente des modules en compression et en torsion de l'ordre de la moitié au double des modules correspondants du capiton plantaire humain.

Selon un mode de réalisation, le gel viscoélastique est un gel silicone auto-adhésif.

Selon un mode de réalisation, la couche de gel viscoélastique est recouverte par une pellicule de protection retirable.

Selon un mode de réalisation, le manchon comprend au moins une pièce de tissu comprenant un mélange de fibres de polyamide et de fibres d'élasthanne.

Selon un mode de réalisation, le manchon est réversible et apte à être utilisé à l'endroit avec la couche de gel viscoélastique disposée intérieurement et apte à être utilisé à l'envers avec la couche de gel viscoélastique disposée extérieurement.

Selon un mode de réalisation, le manchon comprend au moins en une zone de sa face interne ou externe deux couches de gel viscoélastique superposées.

Selon un mode de réalisation, le manchon comprend deux couches de gel viscoélastique combinées par inclusion, l'une étant de plus faible surface et incluse dans l'autre.

La présente invention concerne également un procédé de fabrication d'un manchon de protection ou de soin de doigts, orteils ou autres parties du corps, comportant les étapes consistant à assembler par soudure deux pans de tissu comprenant des fibres thermoplastiques en appliquant des pannes chauffantes à la surface d'un premier pan de tissu selon des intervalles réguliers correspondant alternativement à une demi-circonférence de manchon et à un espacement de découpe des manchons, et fixer au moins une plaque de gel viscoélastique sur la surface libre du second pan de tissu, en recouvrant les emplacements se trouvant au droit des zones de soudure.

Selon un mode de réalisation, le procédé comprend une étape consistant à découper le complexe formé par les pans de tissu assemblés en suivant des traits de coupe se trouvant entre deux lignes de soudure, afin d'obtenir des portions de complexe en forme de tubes aplatis pouvant ensuite être sectionnées en manchons individuels de longueur voulue.

Selon un mode de réalisation, les pannes chauffantes ont une largeur de l'ordre de quelques dixièmes de millimètres à quelques millimètres.

Selon un mode de réalisation, les intervalles correspondant aux espacements de découpe des manchons ont une largeur de l'ordre de quelques millimètres.

La présente invention concerne également un procédé de fabrication d'un manchon de protection ou de soin de doigts, orteils ou autres parties du corps, comprenant les étapes consistant à agencer au moins une plaque de gel viscoélastique sur une face d'un pan de tissu comprenant des fibres thermoplastiques, sans recouvrir de gel deux bords opposés du pan de tissu, replier le pan de tissu de manière que chaque bord se divise en deux parties se trouvant en regard, et souder ensemble les parties respectives de chaque bord qui se trouvent en regard.

Selon un mode de réalisation, les zones de soudure des parties en regard sont très proche des limites de la plaque de gel.

Ces objets, caractéristiques et avantages de la présente invention ainsi que d'autres seront exposés plus en détail dans la description suivante de deux modes de réalisation de manchons selon l'invention et de procédés de fabrication de ces manchons, faite à titre non limitatif en référence aux dessins annexés parmi lesquels :
- la figure 1 est une vue en perspective d'un premier type de manchon selon l'invention, représenté à plat avant retournement,
- la figure 2 est une vue en coupe du manchon de la figure 1 après retournement,
- les figures 3a et 3b illustrent deux utilisations du manchon de la figure 1,
- les figures 4 et 5 illustrent un procédé de fabrication du manchon de la figure 1,
- les figures 6 et 7 illustrent un procédé de fabrication d'un second type de manchon selon l'invention,
- la figure 8 est une vue en perspective du second type de manchon selon l'invention, et
- les figures 9 et 10 représentent deux variantes de réalisation de manchons selon l'invention, applicables aux manchons du premier ou du second type.

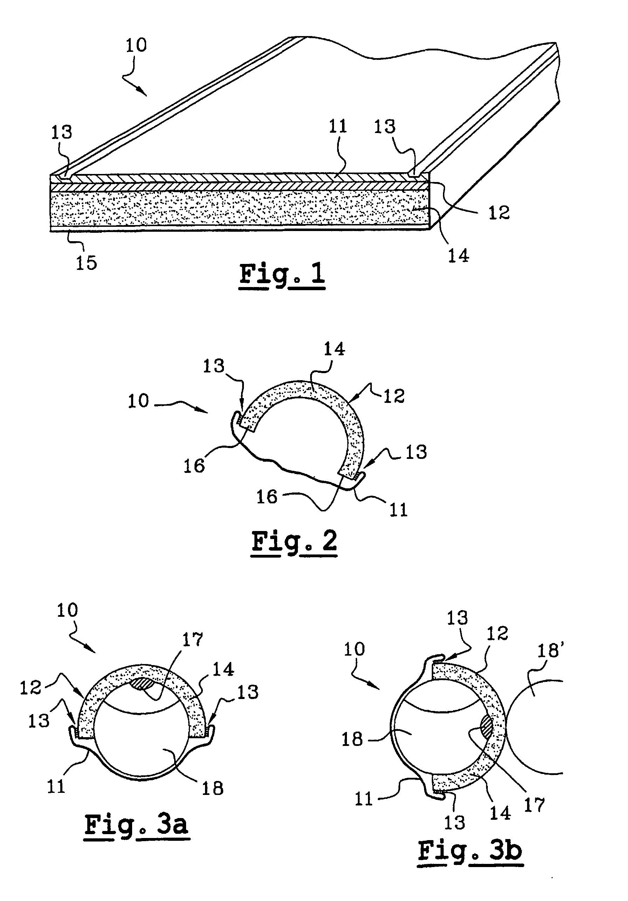

La figure 1 est une vue en perspective d'un premier type de manchon 10 selon l'invention, représenté à plat avant retournement. Selon un premier aspect de la présente invention, le corps du manchon 10 est constitué ici par deux pièces de tissu 11, 12 assemblées par thermosoudage, les pièces de tissu 11, 12 comprenant à cet effet des fibres thermoplastiques. De plus, au moins une des pièces de tissu, de préférence les deux, comprend en outre des fibres élastiques conférant au manchon 10 l'élasticité désirée. Ainsi, de préférence, chaque pièce de tissu comprend un mélange de fibres élastiques et de fibres thermoplastiques. Les fibres élastiques sont de préférence constituées d'élasthanne et les fibres thermoplastiques sont constituées de polyamide. Par exemple, les pièces de tissu comprennent chacune un mélange de fibres de polyamide et d'élasthanne en proportions respectives de 70% et de 30%. De façon avantageuse, le tissu de chaque pièce 11, 12 est fin et son grammage est de préférence inférieur à 250 g/m².

Selon le mode de réalisation illustré en figure 1, les deux pièces de tissu 11, 12 sont de forme rectangulaire et sont rigoureusement superposées. L'assemblage des deux pièces de tissu est assuré par deux lignes de soudure 13 réalisées du côté de la face libre de la pièce de tissu 11, longeant chacune un grand côté de la double pièce de tissu. Comme on le verra plus en détail dans ce qui suit, la présence de fibres thermoplastiques dans chacune des pièces de tissu, notamment des fibres polyamide, permet une réalisation aisée de ces zones de soudure par un simple échauffement localisé entraînant une fusion des fibres.

Selon un autre aspect de la présente invention, l'une des deux pièces de tissu, ici la pièce 12, présente sur sa face libre se trouvant à l'opposée des lignes de soudure 13 une garniture 14 constituée par un gel silicone viscoélastique. Cette garniture 14 se présente de façon avantageuse sous la forme d'une couche de gel uniforme d'une épaisseur de quelques dixièmes de millimètre à quelques millimètres, assemblée par collage sur la face libre de la pièce de tissu 12. La couche de gel silicone 14 couvre de préférence la totalité de la surface de la pièce de tissu 12 et couvre notamment des emplacements se trouvant au droit des lignes de soudure 13.

La présente invention se fonde ici sur le fait que certains gels silicone ont des propriétés de viscoélasticité remarquables, similaires aux propriétés mécaniques de certains tissus cutanés humains. Parmi ces gels silicone viscoélastiques, les compositions de mélanges polymères silicones décrites dans le brevet FR-2 712 487, auquel on se référera pour des détails de formulation et d'élaboration, ont la particularité surprenante de reproduire les propriétés mécaniques du capiton plantaire naturel et de présenter notamment des valeurs similaires de modules en compression et en torsion. Des gels silicone ayant de telles formulations sont commercialisés par la demanderesse sous l'appellation EPITHELIUM 26®. D'autres formulations permettent en outre d'obtenir, parfois au détriment de la similarité des modules mécaniques en compression et en torsion, des propriétés d'adhérence intrinsèque du gel silicone. De tels gels silicone sont commercialisés par la demanderesse sous l'appellation EPITHELIUM 27 et EPIHELIUM 27+. Ils présentent des qualités de contact exceptionnelles et sont des répartiteurs de charge hors pair susceptibles de donner des résultats remarquables en confort et en prévention ainsi qu'en traitement de certaines affections, en particulier des hyperkératoses. En pratique, la conformité de ces gels peut être vérifiée en contrôlant les modules en compression et en torsion dont les valeurs nominales sont de l'ordre de 4.10³ N/m² et de 4.10³ N/m² à 15.10³ N/m², respectivement. Une tolérance de 50% par rapport aux valeurs limites de ces modules peut être acceptée sans perdre les avantages de ces gels pour l'application médicale ou paramédicale visée ici.

Ainsi, selon un mode de réalisation préféré de la présente invention, le gel formant la garniture 14 du manchon 10 est un gel silicone viscoélastique dont les propriétés mécaniques sont de l'ordre de celles du capiton plantaire humain, le gel ayant notamment des modules en compression et en torsion de l'ordre de la moitié au double des modules correspondants du capiton plantaire. Il s'agit par exemple du gel silicone susmentionné commercialisé sous l'appellation EPITHELIUM 26.

Dans une variante de réalisation, le gel silicone présente, outre des propriétés mécaniques similaires au capiton plantaire humain, la propriété d'être adhérent intrinsèquement par simple effet de contact avec la peau, le gel pouvant se décoller et se recoller quasi indéfiniment. Il s'agit par exemple du gel silicone commercialisé sous l'appellation EPITHELIUM 27 OU EPITHELIUM 27+.

Comme on le verra plus loin, deux types de gel silicone peuvent également être combinés dans un manchon selon l'invention.

De préférence, une pellicule de protection 15 retirable est appliquée sur la surface libre de la couche de gel silicone 14 afin de préserver la propreté de sa surface. La pellicule 15 est par exemple en matière plastique, notamment en polyéthylène.

Sur la figure 1, le manchon 10 selon l'invention se présente ainsi à plat, les deux pièces de tissu 11 et 12 étant disposées l'une contre l'autre avec la couche de gel silicone 14 orientée vers l'extérieur. Le manchon se trouve ainsi assemblé à l'envers avec les lignes de soudure 13 apparentes. Il est possible d'utiliser le manchon tel quel en insérant un doigt ou un orteil entre les deux pièces de tissu 11 et 12, la couche de gel 14 se trouvant alors orientée vers l'extérieur pour venir au contact et protéger un doigt ou un orteil voisin.

Le manchon 10 peut également être retourné (retroussé) après avoir retiré la pellicule de protection 15. Celui-ci se présente alors arqué sous une forme quelque peu tubulaire comme illustré par la vue en coupe de la figure 2. Plus particulièrement, le manchon 10 obtenu comprend une portion de tissu 12 sensiblement semi-tubulaire portant le gel silicone 14 sur sa face interne, et une portion de tissu 11 formant l'autre moitié du manchon, ayant un aspect relâché tant que le manchon n'est pas enfilé sur un doigt ou un orteil. Le manchon étant retourné, la couche de gel de silicone 14 se trouve orientée vers l'intérieur du manchon.

Etant donné que selon le mode d'exécution préféré de l'invention la couche de gel 14 couvre toute la surface interne de la pièce 12, la couche de gel 14 couvre ainsi, vue de l'intérieur du manchon, les zones de soudure 13 des pièces de tissu 11, 12, qui se trouvent disposées extérieurement et en retrait par rapport aux limites 16 de la couche de gel 14.

Sur la figure 3a, le manchon 10 est représenté retourné et enfilé autour d'un doigt ou d'un orteil 18 affecté d'une lésion 17, par exemple un cor dorsal. Les pièces de tissu 11, 12 coopèrent pour le maintien élastique du manchon autour de l'orteil 18. Ici, la déformation du manchon 10 et la tension de maintien élastique sont essentiellement supportées par la pièce de tissu 11 qui est exempte de gel, l'autre pièce de tissu 12 ayant essentiellement une fonction de support et de maintien du gel 14 contre la partie de l'orteil 18 présentant la lésion 17. Le tissu de la pièce 11 est donc préférentiellement élastique tandis que la pièce 12 peut l'être également, mais dans une moindre nécessité.

Une fois agencé autour de l'orteil 18, la couche de gel silicone viscoélastique 14 couvre alors sensiblement la moitié de la surface interne du manchon 10 et les deux pièces de tissu 11 et 12 forment deux demi-cylindres en suivant le mode de réalisation le plus simple dans lequel les pièces de tissu 11, 12 ont les mêmes dimensions, diverses variantes pouvant bien entendu être prévues. Comme on l'a déjà indiqué, les zones de soudure 13 se trouvent disposées extérieurement et en retrait par rapport aux limites de la couche de gel 14, de sorte que cette dernière s'interpose entre l'orteil 18 et les surépaisseurs dues à l'assemblage et au retournement des pièces de tissu 11, 12.

Toujours à titre d'exemple, la figure 3b représente un manchon enfilé autour d'un doigt ou un orteil 18 affecté d'un cor inter-digital 17 se trouvant en regard d'un orteil voisin 18'. Dans ce cas, le manchon est orienté de manière que les zones de soudure 13 des pièces de tissu 11, 12 ne se trouvent pas dans l'espace inter-digital.

De façon générale, il apparaît au regard de ce qui précède qu'un manchon selon l'invention répond de façon avantageuse aux objectifs fixés au préambule. Le manchon présente un encombrement minime, assurant un grand confort des doigts ou des orteils, les pièces de tissu étant particulièrement fines et la couche de gel silicone de protection ayant une épaisseur limitée, de préférence de l'ordre du millimètre. Ceci résulte des excellentes caractéristiques mécaniques des gels silicone viscoélastiques, tel que les gels EPITHELIUM 26, EPITHELIUM 27 et EPITHELIUM 27+ mentionnés à titre d'exemple non limitatif. Le manchon offre également une excellente tenue en place, la pièce de tissu exempte de gel conservant l'intégralité de son élasticité. En outre, une telle réserve d'élasticité permet de limiter le nombre de tailles de manchons et ainsi de couvrir toutes les tailles de doigts ou d'orteils à partir de seulement trois tailles standard. Egalement, les zones de soudure et de retournement des pièces de tissu du manchon ne provoquent pas d'inconfort ou d'effet pervers au niveau de la zone du doigt ou de l'orteil à protéger, car le gel silicone viscoélastique s'intercale entre elles. Enfin, le manchon est de préférence constitué de matériaux qui le rendent anallergique, non-irritant et non-cytotoxique. En outre, ces matériaux permettent avantageusement au manchon d'être lavable et réutilisable.

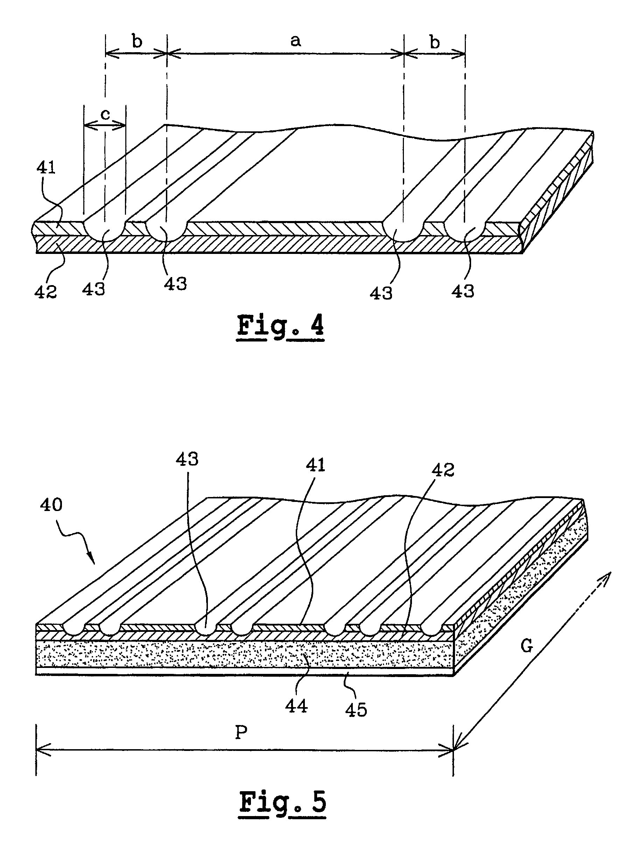

Les figures 4 et 5 illustrent un procédé de fabrication collective du manchon qui vient d'être décrit. Comme illustré en figure 4, l'étape initiale du procédé selon l'invention consiste à assembler deux pans de tissu 41, 42 composés chacun, de préférence, de fibres élastiques et de fibres thermoplastiques. Comme on le voit en figure 5, la largeur P des pans de tissu 41, 42 est suffisamment grande pour permettre de réaliser en parallèle plusieurs lignées de manchons de largeurs identiques ou différentes. La longueur G des pans de tissu est déterminée par les nécessités de la production, qui peut d'ailleurs se faire en continu.

La figure 4 montre le détail de l'assemblage des pans de tissu 41, 42. Les deux pans sont disposés à plat, l'un sur l'autre, et forment un pan de tissu 40 de fabrication collective de grande largeur sur lequel sont appliquées des électrodes ou des pannes chauffantes semblables à celles des fer à souder. L'échauffement et l'empreinte de chaque panne provoque une fusion localisée des fibres thermoplastiques des deux pans de tissu et forme des lignes de soudure en creux 43.

Les pannes chauffantes peuvent être linéaires ou être ponctuelles, les lignes de soudure 43 étant dans ce cas réalisées avec un déplacement linéaire relatif des pannes et de la surface du tissu. Elles sont positionnées à intervalles réguliers a, b correspondant respectivement à la largeur ou demi-circonférence de chaque manchon et à un espacement entre deux manchons sur le pan 40 de fabrication collective. On réalise ainsi des lignes de soudure 43 parallèles et appariées. L'espacement b entre deux lignes creuses 43 atteint quelques millimètres, typiquement 2 mm à 5 mm. De préférence, les lignes creuses 43 formées par la forme des pannes chauffantes ont une largeur c de l'ordre du millimètre ou de quelques millimètres, typiquement de l'ordre de 0,5 mm à 3 mm. Il est préférable que la somme de l'espacement b entre deux lignes de soudure et de la largeur c d'une ligne de soudure 43 n'excède pas 6 mm car cette dimension détermine l'excédent de tissu que la couche de gel devra isoler du doigt ou de l'orteil à protéger. Une telle étape d'assemblage fournit un ensemble de tubes de tissu aplatis et attachés. La surface sans soudure du tissu 42, se trouvant sous le tissu 41 relativement au sens d'application des pannes, est parfaitement plane.

L'étape suivante du procédé selon l'invention, illustrée en figure 5, consiste à coller une plaque de gel silicone viscoélastique 44 contre la surface plane intacte du pan de tissu 42. De préférence, la face libre de la plaque de gel silicone 44 est protégée par un film plastique 45 retirable, tel qu'un film polyéthylène, avant de procéder au collage. A l'issue de l'étape de collage, on obtient donc un ensemble multicouche tissu/tissu/gel/film comprenant une rangée de manchons aplatis à l'envers et non détachés. L'ensemble multicouche peut alors être découpé suivant des traits de coupe se trouvant dans les espacements b entre les paires de lignes de soudure 43, afin de détacher des bandes de manchons aplatis. Ensuite, ces bandes peuvent être elles-mêmes sectionnées pour former des manchons de longueur voulue.

Ce procédé de fabrication de manchons selon l'invention a l'avantage de comprendre des étapes simples de réalisation à plat des manchons et permet de fabriquer collectivement des manchons ayant un prix de revient réduit.

Toutefois, d'autres variantes et modes de réalisation de la présente invention sont également envisageables.

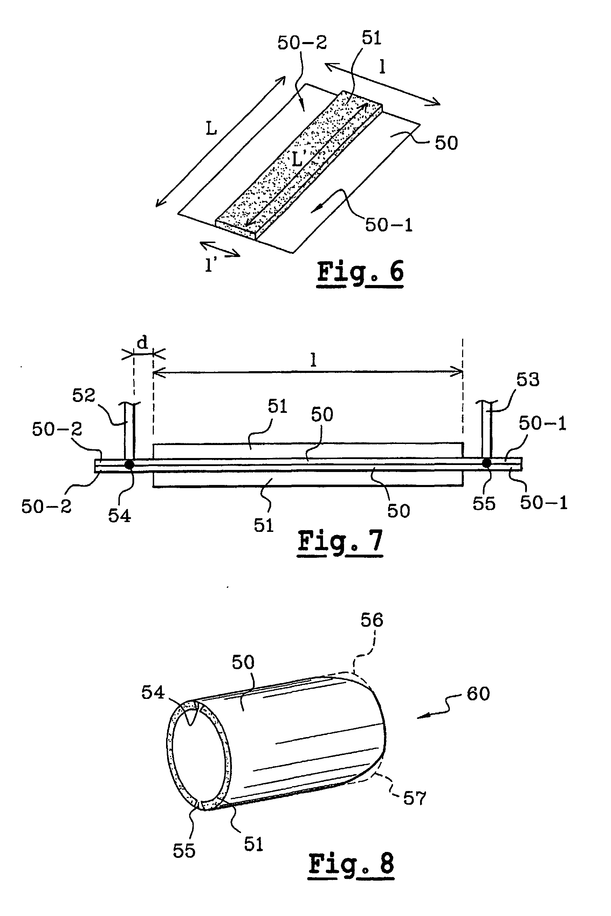

Ainsi, les figures 6 et 7 représentent un procédé de fabrication d'un second type de manchon, à bout fermé, réalisé par pliage d'une pièce de tissu.

Comme représenté en figure 6, une pièce de tissu rectangulaire 50 de longueur L et de largeur 1 comprenant des fibres élastiques et thermoplastiques est posée à plat sur un support. La pièce 50 reçoit sur sa face libre une bande de gel silicone viscoélastique 51 rectangulaire assemblée par collage. La bande de gel 51 présente une longueur L' de préférence égale à L et une largeur l' inférieure à 1, et est agencée sur la pièce de tissu 50 de façon centrée pour laisser apparaître deux bords 50-1, 50-2 de la pièce 50 qui ne sont pas recouverts de gel. La bande de gel 51 est de préférence recouverte par une pellicule de protection retirable (non représentée).

A l'étape illustrée en figure 7, la pièce de tissu 50 est repliée en deux parties égales dans le sens de sa largeur 1 de manière que les moitiés des bords 50-1, 50-2 se trouvent respectivement face à face. De préférence, le repliement est effectué de manière que la bande de gel 51 se trouve à l'extérieur du manchon en cours de formation. Les deux moitiés des bords de tissu 50-1, 50-2 sont ensuite pincées et soudées l'une contre l'autre au moyen de pannes chauffantes 52, 53 pour former des lignes de soudure 54, 55 vues en coupe sur la figure 7. Les pannes 52, 53 sont étroites, par exemple quelques dixièmes de millimètre, pour l'obtention de lignes de soudure 54, 55 de faible largeur. Comme précédemment, les lignes de soudure 54, 55 peuvent être faite en continu ou point à point avec déplacement relatif de des pannes 52, 53 et de la pièce de tissu 50. L'utilisation de pannes présentant une longueur dans le plan de soudure correspondant à l'étendue des lignes de soudure 54, 55 peut également être prévue. La distance d entre les bandes de soudure 54, 55 et les bords de la bande de gel 51 est de préférence très faible, par exemple de l'ordre de 0,5 mm. Les parties des bords de tissu 50-1, 50-2 qui s'étendent au-delà des lignes de soudure 54, 55 sont ensuite sectionnées. Les deux coins de tissu se trouvant à l'extrémité des lignes de soudure 54, 55 du côté de l'extrémité fermée du manchon peuvent également être sectionnés, pour obtenir à l'extrémité fermée du manchon des coins chanfreinés 56, 57 représentés en figure 8.

Les caractéristiques, notamment la nature et les épaisseurs des matériaux utilisés dans ce mode de réalisation sont choisies conformément à l'enseignement exposé plus haut.

La figure 8 représente le manchon 60 obtenu par ce procédé, après retournement de celui-ci. Le manchon 60 se distingue du manchon 10 décrit plus haut par le fait que la couche de gel 51 recouvre pratiquement toute sa surface intérieure à l'exception des zones de jonction des bords 50-1, 50-2 de la pièce de tissu 50, où se trouvent les lignes de soudure 54, 55. Toutefois, l'épaisseur de la bande de gel 51 empêche l'excroissance formée par les zones de jonction de venir frotter contre la peau.

Le procédé qui vient d'être décrit se prête bien, comme le précédent, à une fabrication collective de manchons. De plus, la bande de tissu 50 et la bande de gel 51 représentées en figure 6 peuvent être longues et être découpées à la longueur voulue après l'étape de soudage illustré en figure 7.

La présente invention est bien entendu susceptible de diverses autres variantes de réalisation. Par exemple, deux bandes de tissu semblables à celle représentée en figure 6, chacune étant pourvue d'une bande de gel, peuvent être assemblées l'une contre l'autre et soudées à leurs extrémités pour obtenir un manchon comprenant deux demi-cylindres pourvus chacun d'une couche de gel. Egalement, la pièce de tissu 50 décrite ci-dessus peut être repliée longitudinalement et avoir ses bords opposés 50-1, 50-2 soudés ensemble pour former un manchon tubulaire, qui peut ensuite être sectionné en plusieurs manchons tubulaires de moindre longueur.

Par ailleurs, la couche de gel de silicone recouvrant tout ou partie de la face interne ou externe d'un manchon selon l'invention (selon le sens d'utilisation du manchon) peut comprendre une combinaison de plusieurs bandes de gel silicone, de même nature ou non.

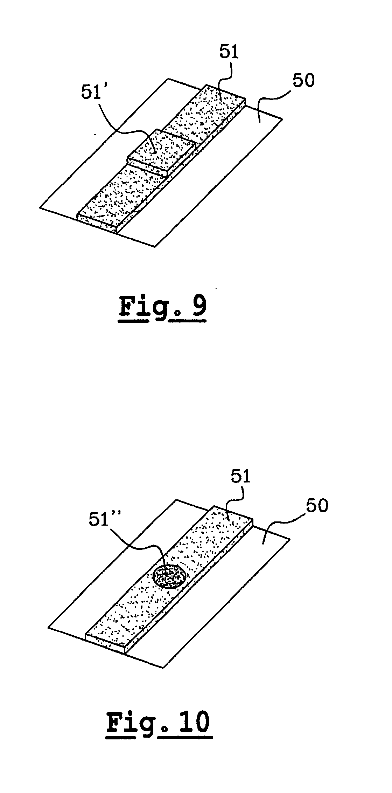

Pour fixer les idées, la figure 9 représente une bande de tissu 50 identique à celle de la figure 6, portant une bande de gel 51 qui est recouverte localement, dans sa partie centrale, par une deuxième couche de gel 51' de moindre longueur, assemblée par collage. La couche de gel supplémentaire 51' est par exemple de forme carrée et d'une épaisseur de l'ordre de 0,5 mm à 1,5 mm.

La bande de gel 51 et la couche supplémentaire 51' peuvent être de même formulation, par exemple en EPITHELIUM 26. Dans ce cas, la couche 51' joue le rôle d'un simple renfort qui offre une plus grande protection de l'extrémité de l'orteil ou du doigt face aux sollicitations extérieures. Notamment, la couche 51' est particulièrement utile contre les microtraumatismes de la marche qui provoquent des ongles bleus.

La bande de gel 51 peut également être en EPITHELIUM 26 et la couche supplémentaire 51' en EPITHELIUM 27, ou inversement. Dans ce cas, les propriétés auto-adhésives de l'EPITHELIUM 27 s'associent aux qualités de répartition de charge de l'EPITHELIUM 26 pour inhiber totalement les frottements à la surface de la peau.

Enfin, encore une variante de réalisation illustrée en figure 10 consiste à prévoir dans la partie centrale de la bande de silicone 51 agencée sur la pièce de tissu 50, une inclusion d'une pièce de silicone 51" d'un type différent, par exemple une inclusion d'EPITHELIUM 26 dans une bande d'EPITHELIUM 27 ou inversement.

## Revendications

1. Manchon (10, 60) de protection ou de soin de doigts, orteils ou autres parties du corps, **caractérisé en ce qu'**il comprend au moins une pièce de tissu (11, 12, 50) comprenant des fibres thermoplastiques, assemblée par soudage le long de ses bords pour former tout ou partie du corps du manchon, et au moins une couche de gel viscoélastique (14, 50, 51', 51") agencée sur une face de la pièce de tissu.

2. Manchon (10) selon la revendication 1, **caractérisé en ce qu'**il comprend deux pièces (11, 12) de tissu soudées (13) ensemble le long de deux bords, l'une des pièces de tissu (12) portant la couche de gel viscoélastique (14), les zones de soudure (13) des deux pièces de tissu étant en retrait des limites (16) de la couche de gel.

3. Manchon selon la revendication 2, **caractérisé en ce qu'**au moins une des deux pièces de tissu (11) comprend des fibres élastiques.

4. Manchon (60) selon la revendication 1, **caractérisé en ce qu'**il comprend une pièce de tissu repliée (50) comprenant des fibres élastiques et des fibres thermoplastiques, ayant deux bords opposés (50-1, 50-2) soudés et portant sur l'une de ses faces la couche de gel viscoélastique (50, 50', 50").

5. Manchon selon l'une des revendications 1 à 4, **caractérisé en ce que** le gel viscoélastique est un gel silicone.

6. Manchon selon la revendication 5, **caractérisé en ce que** le gel silicone présente des modules en compression et en torsion de l'ordre de la moitié au double des modules correspondants du capiton plantaire humain.

7. Manchon selon l'une des revendications 1 à 6, **caractérisé en ce que** le gel viscoélastique est un gel silicone auto-adhésif.

8. Manchon selon l'une des revendications 1 à 7, **caractérisé en ce que** la couche de gel viscoélastique (14) est recouverte par une pellicule de protection (15) retirable.

9. Manchon selon l'une des revendications 1 à 8, **caractérisé en ce qu'**il comprend au moins une pièce de tissu comprenant un mélange de fibres de polyamide et de fibres d'élasthanne.

10. Manchon selon l'une des revendications 1 à 9, **caractérisé en ce qu'**il est réversible et apte à être utilisé à l'endroit avec la couche de gel viscoélastique disposée intérieurement et apte à être utilisé à l'envers avec la couche de gel viscoélastique disposée extérieurement.

11. Manchon selon l'une des revendications 1 à 10, **caractérisé en ce qu'**il comprend au moins en une zone de sa face interne ou externe deux couches (51, 51') de gel viscoélastique superposées.

12. Manchon selon l'une des revendications 1 à 10, **caractérisé en ce qu'**il comprend deux couches de gel viscoélastique (51, 51') combinées par inclusion, l'une étant de plus faible surface et incluse dans l'autre.

13. Procédé de fabrication d'un manchon (10) de protection ou de soin de doigts, orteils ou autres parties du corps, **caractérisé en ce qu'**il comprend les étapes consistant à :
- assembler par soudure deux pans (41, 42) de tissu comprenant des fibres thermoplastiques en appliquant des pannes chauffantes à la surface d'un premier pan de tissu selon des intervalles réguliers (a, b) correspondant alternativement à une demi-circonférence de manchon et à un espacement de découpe des manchons, et
- fixer au moins une plaque de gel viscoélastique (44) sur la surface libre du second pan de tissu, en recouvrant les emplacements se trouvant au droit des zones de soudure.

14. Procédé selon la revendication 13, **caractérisé en ce qu'**il comprend une étape suivante consistant à découper le complexe (40) formé par les pans de tissu assemblés en suivant des traits de coupe se trouvant entre deux lignes de soudure (43), afin d'obtenir des portions de complexe (40) en forme de tubes aplatis pouvant ensuite être sectionnées en manchons individuels de longueur voulue.

15. Procédé selon l'une des revendications 13 et 14, **caractérisé en ce que** les pannes chauffantes ont une largeur (c) de l'ordre de quelques dixièmes de millimètres à quelques millimètres.

16. Procédé selon l'une des revendications 13 à 15, **caractérisé en ce que** les intervalles correspondant aux espacements de découpe des manchons ont une largeur (b) de l'ordre de quelques millimètres.

17. Procédé de fabrication d'un manchon (60) de protection ou de soin de doigts, orteils ou autres parties du corps, **caractérisé en ce qu'**il comprend les étapes consistant à :
- agencer au moins une plaque de gel viscoélastique (51) sur une face d'un pan de tissu (50) comprenant des fibres thermoplastiques, sans recouvrir de gel deux bords opposés (50-1, 50-2) du pan de tissu,
- replier le pan de tissu de manière que chaque bord se divise en deux parties se trouvant en regard, et souder (54, 55) ensemble les parties respectives de chaque bord (50-1, 50-2) qui se trouvent en regard.

18. Procédé selon la revendication 17, dans lequel les zones de soudure (54, 55) des parties en regard sont très proche des limites de la plaque de gel (51).

## Patentansprüche

1. Schutz- oder Pflegehülse (10, 60) für Finger, Zehen oder andere Teile des Körpers, **dadurch gekennzeichnet, dass** sie zumindest ein Textilteil (11, 12, 50) aufweist, welches thermoplastische Fasern aufweist, welches Textilteil durch Schweißen entlang seiner Kanten zusammengefügt ist, um zumindest einen Teil des Körpers der Hülse zu bilden, und zumindest eine Schicht aus viskoelastischen Gel (14, 50, 51', 51"), die auf eine Fläche des Textilteils aufgebracht ist.

2. Hülse (10) nach Anspruch 1, **dadurch gekennzeichnet, dass** sie zumindest zwei Textilteile (11, 12) aufweist, die entlang zweier Kanten verschweißt (13) sind, wobei eines (12) dieser Textilteile die Schicht aus viskoelastischem Gel (14) trägt, und wobei die Schweißbereiche (13) der beiden Textilteile von den Grenzen (16) der Gelschicht zurückweichen.

3. Hülse nach Anspruch 2, **dadurch gekennzeichnet, dass** zumindest eines (11) der beiden Textilteile elastische Fasern aufweist.

4. Hülse (60) nach Anspruch 1, **dadurch gekennzeichnet, dass** sie ein zusammengefaltetes Textilteil (50) aufweist, welches elastische Fasern und thermoplastische Fasern aufweist, mit zwei gegenüberliegenden, verschweißten Kanten (50-1, 50-2) und mit der Schicht aus viskoelastischem Gel (50, 50', 50") auf einer seiner Flächen.

5. Hülse nach einem der Ansprüche 1 bis 4, **dadurch gekennzeichnet, dass** das viskoelastische Gel ein Silikongel ist.

6. Hülse nach Anspruch 5, **dadurch gekennzeichnet, dass** das Silikongel einen Kompressions- und Torsionsmodul aufweist in der Größenordnung zwischen der Hälfte und dem Doppelten der entsprechenden Module des menschlichen Fußsohlenpolsters (capiton plantaire) hat.

7. Hülse nach einem der Ansprüche 1 bis 6, dadurch dass das viskoelastische Gel ein selbstklebendes Silikongel ist.

8. Hülse nach einem der Ansprüche 1 bis 7, **dadurch gekennzeichnet dass** die Schicht aus viskoelastischem Gel (14) mit einer Schutzhaut (15) bedeckt ist, die abziehbar ist.

9. Hülse nach einem der Ansprüche 1 bis 8, **dadurch gekennzeichnet, dass** sie zumindest ein Textilteil aufweist, das eine Mischung aus Polyamidfasern und Elastanfasern aufweist.

10. Hülse nach einem der Ansprüche 1 bis 9, **dadurch gekennzeichnet, dass** sie reversibel ist und so verwendet werden kann, dass die viskoelastische Gelschicht innen angeordnet ist, und auch so verwendet werden kann, dass die viskoelastische Gelschicht außen angeordnet ist.

11. Hülse nach einem der Ansprüche 1 bis 10, **dadurch gekennzeichnet, dass** sie zumindest in einem Bereich ihrer inneren Fläche oder äußeren Fläche (2) übereinander angeordnete viskoelastische Gelschichten (51, 51') aufweist.

12. Hülse nach einem der Ansprüche 1 bis 10, **dadurch gekennzeichnet, dass** sie zwei viskoelastische Gelschichten (51, 51') aufweist, die durch Einschluss verbunden sind, wobei die eine eine weichere Oberfläche hat und in der anderen eingeschlossen ist.

13. Verfahren zur Herstellung einer Schutz- oder Pflegehülse (10) für Finger, Zehen oder andere Teile des Körpers, **dadurch gekennzeichnet, dass** das Verfahren die folgenden Schritte aufweist:
- Verbinden zweier Bahnen (41, 42) aus einem Textilmaterial mit thermoplastischen Fasern durch Schweißen, indem erwärmte Finnen auf die Oberfläche einer ersten Textilbahn in regelmäßigen Abständen (a, b) aufgebracht werden, die alternativ dem halben Umfang der Hülse und einem Zwischenraum des Schneidvorgangs der Hülsen entsprechen, und
- Befestigen von zumindest einer Platte aus viskoelastischem Gel (14) auf der freien Oberfläche der zweiten Textilbahn, wodurch die Stellen bedeckt werden, die sich rechts von den Schweißbereichen befinden.

14. Verfahren nach Anspruch 13, **dadurch gekennzeichnet, dass** es einen darauffolgenden Schritt aufweist, der in dem Zerschneiden des Komplexes (40) besteht, der durch die zusammengefügten Textilbahnen gebildet worden ist, folgend den Schnittspuren, die sich zwischen zwei Schweißlinien (43) befinden, um Teile dieses Komplexes (40) in Form von flachen Röhren zu erhalten, die dann in individuelle Hülsen der gewünschten Länge zerschnitten werden können.

15. Verfahren nach einem der Ansprüche 13 und 14, **dadurch gekennzeichnet, dass** die erhitzten Finnen eine Breite (c) in der Größenordnung von einigen Zehntel Millimetern bis hin zu einigen Millimetern haben.

16. Verfahren nach einem der Ansprüche 13 bis 15, **dadurch gekennzeichnet, dass** die Abstände, die den Zwischenräumen des Schneidvorgangs der Hülse entsprechen, eine Breite (b) in der Größenordnung von einigen Millimetern haben.

17. Verfahren zur Herstellung einer Schutz- oder Pflegehülse (60) für Finger, Zehen oder andere Teile des Körpers, **dadurch gekennzeichnet, dass** das Verfahren die folgenden Schritte aufweist:
- Aufbringen von zumindest einer Platte aus viskoelastischem Gel (51) auf eine Fläche einer Textilbahn (50), die thermoplastische Fasern aufweist, ohne zwei gegenüberliegende Kanten (50-1, 50-2) der Textilbahn mit Gel zu bedecken,
- Falten der Textilbahn so, dass jede Kante sich in zwei Teile aufteilt, die sich gegenüberliegen, und Schweißen (54, 55) der jeweiligen Teile jeder Kante (50-1, 50-2) zusammen, die sich gegenüberliegen.

18. Verfahren nach Anspruch 17, in welchem die Schweißbereiche (54, 55) der gegenüberliegenden Teile sehr nahe an den Grenzen der Gelplatte (51) liegen.

## Claims

1. Sleeve (10, 60) for the protection or care of fingers, toes or other parts of the body, **characterised in that** it comprises at least one piece of material (11, 12, 50) comprising thermoplastic fibres, joined along the length of its sides to form all or part of the body of the sleeve, and at least one layer of viscoelastic gel (14, 50, 51', 51") on one side of the piece of material.

2. Sleeve (10) according to claim 1, **characterised in that** it comprises two pieces (11, 12) of material joined together (13) along two sides, one of the pieces of material (12) bearing the layer of viscoelastic gel (14), and the joins (13) of the two pieces of material being apart from the borders (16) of the layer of gel.

3. Sleeve according to claim 2, **characterised in that** at least one of the pieces of material (11) comprises elastic fibres.

4. Sleeve (60) according to claim 1, **characterised in that** it comprises a folded piece of material (50) comprising elastic and thermoplastic fibres, with two joined, opposite edges (50-1, 50-2) and bearing, on one of its sides, the layer of viscoelastic gel (50, 50', 50").

5. Sleeve according to one of claims 1 to 4, **characterised in that** the viscoelastic gel is a silicone gel.

6. Sleeve according to claim 5, **characterised in that** the silicone gel presents modules in compression and torsion to the order of half or double of the corresponding modules of human plantar padding.

7. Sleeve according to one of claims 1 to 6, **characterised in that** the viscoelastic gel is a self-adhesive silicone gel.

8. Sleeve according to one of claims 1 to 7, **characterised in that** the layer of viscoelastic gel (14) is covered with a removable protective film (15).

9. Sleeve according to one of claims 1 to 8, **characterised in that** it comprises at least one piece of material comprising a mix of polyamide and elasthane fibres.

10. Sleeve according to one of claims 1 to 9, **characterised in that** it is reversible and may be used on the right side, with the layer of viscoelastic gel on the inside, or on the reverse side with the layer of viscoelastic gel on the outside.

11. Sleeve according to one of claims 1 to 10, **characterised in that** it comprises, on an area of its internal or external side, at least two superimposed layers (51, 51') of viscoelastic gel.

12. Sleeve according to one of claims 1 to 10, **characterised in that** it comprises two layers of viscoelastic gel (51, 51') combined by inclusion, with one layer having a weaker surface and being embedded in the other.

13. Method of producing a sleeve (10) for the protection or care of fingers, toes or other parts of the body, **characterised in that** it comprises steps consisting of :
- joining together two sections of material (41, 42) comprising thermoplastic fibres, by applying heated plates to the surface of a first section of material at regular intervals (a, b) corresponding alternately to a semi-circle of sleeve and to a space cut out of the sleeves and,
- fixing at least one patch of viscoelastic gel (44) onto the free surface of the second section of material, whilst covering the places at right angles to the joined areas.

14. Method according to claim 13, **characterised in that** it comprises a further step consisting of cutting out the complex (40), formed by the assembled sections of material, by following cutting guides between two joins (43) so that the complex (40) has portions in the form of flattened tubes which may then be sectioned into individual sleeves of a desired length.

15. Method according to one of claims 13 and 14, **characterised in that** the heated plates are of a size (c) in the order of several tens of millimetres to several millimetres.

16. Method according to one of claims 13 to 15, **characterised in that** the intervals corresponding to the cut-out spaces in the sleeves are of a size (b) in the order of several millimetres.

17. Method of producing a sleeve (6) for the protection or care of fingers, toes or other parts of the body, **characterised in that** it comprises steps consisting of :
- applying at least one patch of viscoelastic gel (51) onto a side of a section of material (50) comprising thermoplastic fibres, without the gel covering two opposite edges (50-1, 50-2) of the section of material,
- folding the section of material in such a way that each edge is divided into two facing parts, and joining together (54, 55) the respective parts of each facing edge (50-1, 50-2).

18. Method according to claim 17, in which the joins (54, 55) of the facing parts are very close to the borders of the patch of gel (51).
